# EUROPEAN PATENT APPLICATION

(11) **EP 4 193 944 A1**
(43) Date of publication of application: **14.06.2023**
(21) Application number: 21872669.3
(22) Date of filing: 15.06.2021
(51) Int. Cl.: A61B 18/14, C25D 11/04, C25D 11/18, A61B 18/00, A61B 18/12

(54) **CONDUCTIVE ELECTRODE FOR ELECTROSURGICAL HANDPIECE**

(30) Priority: 22.09.2020 KR 20200122366; 21.05.2021 KR 20210065249
(71) Applicant: Choi, In-Sang, Anyangsi, Gyeonggi-do 14099 (KR)
(72) Inventor: Choi, In-Sang, Anyangsi, Gyeonggi-do 14099 (KR)
(74) Representative: Thun, Clemens
(86) International application number: PCT/KR2021/007449
(87) International publication number: WO 2022/065631

(57) **Abstract**

The present invention relates to a conductive electrode for a monopolar handpiece used for an electrosurgical handpiece and particularly to a conductive electrode for an electrosurgical handpiece, which causes almost no tissue carbonization and does not generate smog.

## Description

### Technical Field

The present disclosure relates to a conductive electrode for a monopolar handpiece used for an electrosurgical handpiece and, more particularly, to a conductive electrode for an electrosurgical handpiece, which causes minimal tissue carbonization and does not generate smog.

### Background Art

Surgical scalpels made of iron have conventionally been used for tissue-incising operations, and are also currently in widespread use. However, in line with high-level development of modern engineering technologies, cutting-edge surgical tools that use energy, such as electricity, lasers, and ultrasonic waves, have appeared.

Energy-based surgical devices operate according to the following principle: energy is appropriately injected into the tissue of a human body such that a change in the tissue occurs, thereby obtaining a surgical effect.

The most widely used energy-based surgical devices among the same are used during electrosurgery, which is a surgical method for incising, excising, or cauterizing the patient's tissue by using high-frequency electric energy.

The human nervous system reacts very sensitively to low-frequency electricity of up to 1,000Hz, and will be stupefied by an electric shock caused by AC current of mains electricity.

Electrosurgery using high-frequency electric energy uses high-frequency electricity of about 200kHz to 5MHz.

Intracellular vibrations are caused by electric energy supplied through electrodes, and the resulting increase in intracellular temperature heats the tissue.

If the intracellular temperature reaches about 60°C, cell death occurs. If heated to 60-99°C, tissue is dried (dehydration) and protein coagulation occur. If the intracellular temperature reaches about 100°C, cells undergoes volume expansion and vaporization. The tissue is excised or cauterized through such a process.

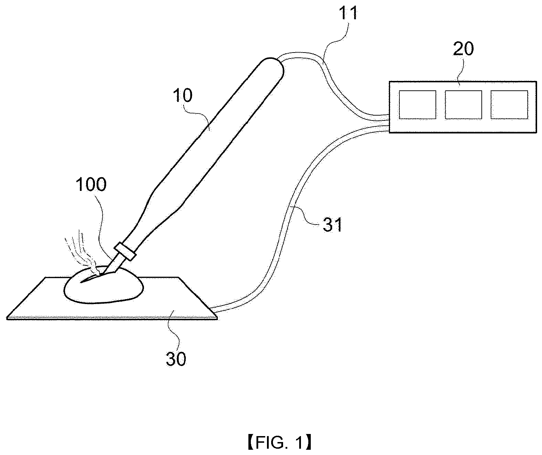

As illustrated in FIG. 1, a monopolar electrosurgical device has a conductive electrode 100 fastened to the front side of a handpiece 10 which is held by a surgeon, and has a ground pad 30 grounded to a patient. The handpiece 10 and the ground pad 30 are connected to a control unit 20 for generating high frequencies through cables 11 and 31, respectively.

As such, electrosurgery uses high-frequency electric currents to excise and coagulate tissue. During excision using an electrosurgical device, heat is generated by tissue excision using high-frequency electric currents, thereby resulting in noticeable coagulation.

Such electrosurgical excision has a problem in that incomplete contact between the conductive electrode and the tissue inevitably destroys the air insulating layer, thereby generating an arc involving high-temperature heat, the tissue is set ablaze by the arc and thus burns, and the tissue carbonizes and contaminates the conductive electrode.

The carbonized tissue abruptly increases the resistance of the contaminated electrode part and block the flow of current. Therefore, the electrode always needs to be polished clean, but there is a problem in that the electrode stained by the carbonized tissue is not easily cleaned.

In addition, smog is generated when the tissue is carbonized by an arc, and the smog reportedly has an adverse influence on the health of surgeons and patients.

### Disclosure of Invention

### Technical Problem

The present disclosure has been made to solve the above-mentioned problems, and it is an aspect of the present disclosure to provide a conductive electrode for an electrosurgical handpiece, wherein contamination of the electrode by carbonized tissue can be reduced, carbonized tissue can be cleaned off the conductive electrode, a patient's burn can be reduced, and the occurrence of smog can be minimized.

### Solution to Problem

In accordance with an aspect of the present disclosure, a conductive electrode for an electrosurgical handpiece is a conductive electrode (100) fastened to a handpiece (10) used for monopolar electrosurgery, and includes: a blade (101) molded in a long plate shape; a plug (103) molded to extend from the blade (101) and to be inserted and fastened to the handpiece (10); an anodized coating layer (104) formed on an outer surface of the blade (101) by performing anodized coating; an edge portion (102) formed on a peripheral part of the blade (101) without performing anodized coating; and a non-adhesive coating layer (105) formed by coating a surface of the anodized coating layer (104).

The non-adhesive coating layer (105) may be a ceramic coating layer or a Teflon coating layer.

In addition, the anodized coating layer (104) may be formed to have a thickness of 30um to 50um.

In addition, the non-adhesive coating layer (105) may be formed to have a thickness of 20um to 40um.

In addition, a non-adhesive coating layer (105) may be formed on a surface of the edge portion (102).

In addition, the blade (101) may be press-molded by using aluminum as a material.

In addition, the edge portion (102) may be formed by anodized-coating the edge portion (102) as well and then removing the anodized coating from the edge portion (102).

### Advantageous Effects of Invention

The conductive electrode 100 of the present disclosure configured as described above is easy to process, is inexpensive, has excellent strength, and has an anodized coating layer 104 and a non-adhesive coating layer 105 formed on the surface thereof such that excellent insulation characteristics and non-stick characteristics are exhibited, thereby reducing contamination of the electrode due to carbonized tissue. In addition, carbonized tissue that has adhered to the conductive electrode 100 can be removed clean, burning of tissue can be reduced, and the occurrence of smog can be minimized.

### Brief Description of Drawings

FIG. 1 illustrates the configuration of a monopolar electrosurgical device.
FIG. 2 is a perspective view of a handpiece having a conductive electrode coupled thereto.
FIG. 3 is an exploded perspective view of the handpiece from which the conductive electrode is separated.
FIG. 4 is a perspective of a conductive electrode according to the present disclosure.
FIG. 5 is a sectional view taken along line A-A in FIG. 4, which illustrates the configuration of the conductive electrode.

### Best Mode for Carrying out the Invention

Hereinafter, the present disclosure will be described in detailed with reference to exemplary embodiments of the present disclosure and the accompanying drawings, based on the assumption that identical reference numerals in the drawings denote identical components.

When it is described in the detailed description of the present disclosure or in the claims that a component "includes" another component, the former component is not to be limited or interpreted as solely being made of the latter component, but is to be understood as being able to encompass other components, unless specifically mentioned otherwise.

As used herein, terms "upper portion", "lower portion", "bottom", "front", "rear", and "below" are used only to facilitate descriptions, and denote the orientation of components as illustrated in the drawings.

A conductive electrode 100 used for a handpiece 10 of an electrosurgical device is configured such that tissue is incised, excised, or cauterized by using high-frequency electric energy supplied to the conductive electrode 100.

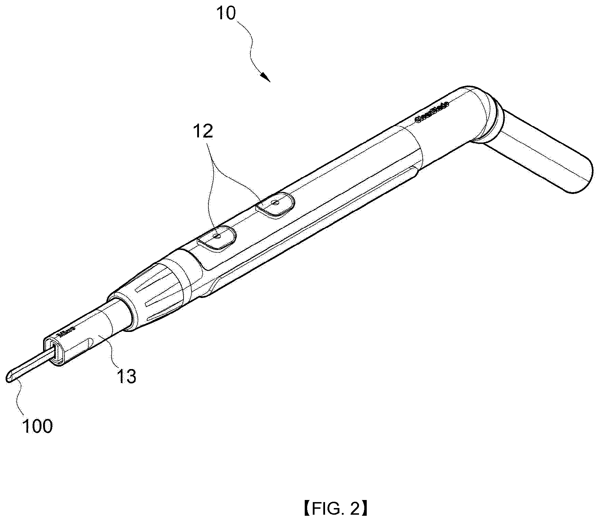

The handpiece 10 of the present disclosure is a monopolar electrosurgical. As illustrated in FIG. 1 and FIG. 2, a conductive electrode 100 is fastened to the front of the handpiece 10 which is held by a surgeon, a ground pad 30 is grounded to a patient, and the handpiece 10 and the ground pad 30 are connected to a control unit 20 for generating high-frequency waves through cables 11 and 31, respectively.

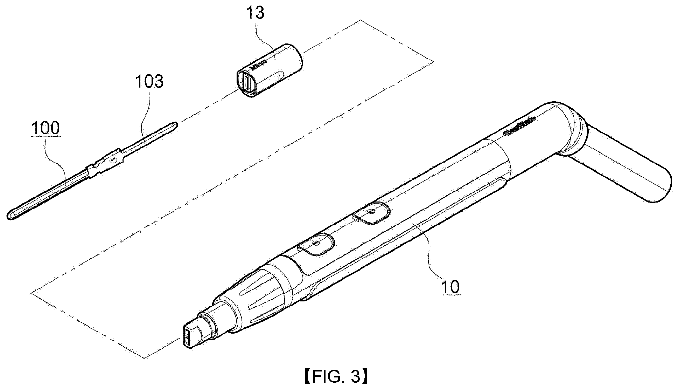

The conductive electrode 100 according to the present disclosure has a plug 103 formed on the rear side of the conductive electrode 100 as illustrated in FIG. 3. The plug 103 is inserted into a front holder 13 of the handpiece 10. High-frequency electric energy generated by the control unit 20 is supplied to the conductive electrode 100 through the cable 11.

The conductive electrode 100 is fastened to the handpiece 10 through the holder 13 such that the conductive electrode 100 can be replaced and used for the handpiece 10.

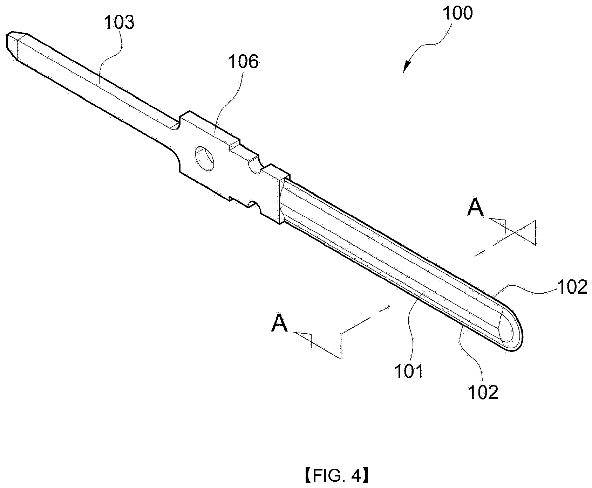

As illustrated in FIG. 4, the conductive electrode 100 largely includes a blade 101 and a plug 103. If the conductive electrode 100 is fastened to the handpiece 10 through the holder 13, the blade 101 part of the conductive electrode 100 is exposed to the outside as in FIG. 2, and the plug 103 is inserted into the handpiece 10 so as to supply electric energy to the blade 101.

An operation is enabled by high-frequency electric energy transferred to the blade 101 of the conductive electrode 100 according to the present disclosure. the blade 101 of the conductive electrode 100 is shaped in a long plate shape so as to facilitate incising, excising, and cauterizing of tissue. Tissue is incised by an edge portion 102, which corresponds to a peripheral part of the blade 101 having a long plate shape.

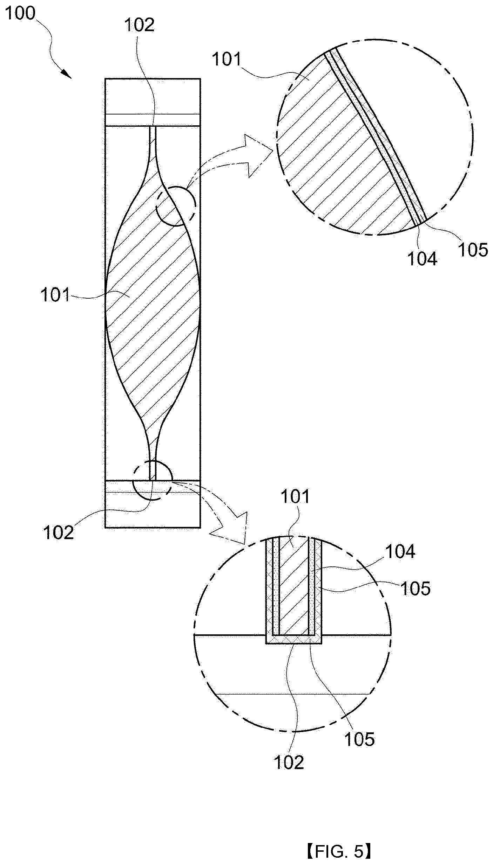

The conductive electrode 100 according to the present disclosure has a blade 101 formed in a plate shape as illustrated in FIG. 4 and FIG. 5, and has a plug 103 formed on the rear side of the blade 101 so as to extend backwards so as to be fastened/fixed to the handpiece 10 and to establish electric connection with the blade 101, thereby supplying electric energy to the blade 101.

As described above, the conductive electrode 100 is inserted and fastened to the holder 13. As illustrated in FIG. 4, the conductive electrode 100 preferably has stoppers 106 formed to protrude from the upper and lower portions thereof, respectively, such that, when the conductive electrode 100 is inserted into the holder 13, the stoppers 106 engage with the holder 13, thereby holding the conductive electrode 100.

In addition, the blade 101 has an edge portion 102 formed on a periphery so as to incise and excise tissue.

The blade 101 is formed to have a streamlined section so as to facilitate penetration into tissue, as illustrated in FIG. 5, and has an edge portion 102 formed on a periphery thereof.

The conductive electrode 100 of the present disclosure is made of aluminum.

Aluminum is characterized in that, compared with other metals commonly used for medical purposes (for example, tungsten, stainless steel), the same is inexpensive, easy to process, and has a high level of strength during post-processing.

Particularly, aluminum is more ductile than other metals and thus can be easily molded through pressing. This is advantageous in that the number of manufacturing processes can be substantially reduced.

High-frequency energy for electrosurgery is applied to the blade 101 of the conductive electrode 100 made of aluminum, and the surface of the blade 101 is anodized to increase the surface strength of the blade 101 and to insulate side surfaces of the blade 101.

Anodizing is for the purpose of increasing the surface rigidity of aluminum. By electrolyzing a metal with a diluted acid liquid through an anode, oxygen generated at the anode form an oxide film (aluminum oxide, Al₂O₃) having strong affinity to the base material.

According to the present disclosure, the surface of the blade 101 is anodized so as to form an anodized coating layer 104 having a thickness of 30um to 50pm as in FIG. 5.

No anodized coating layer 104 is formed on the edge portion 102 of the blade 101, which performs the function of incising and excising tissue during electrosurgery.

The edge portion 102 may be excluded when performing anodized coating, but preferably, the edge portion 102 is also anodized/coated, and the anodized coating layer 104 of the edge portion 102 is removed in a process for removing the anodized coating layer of the edge portion 102 through a post-processing such as polishing.

Tissue is incised and excised by high-frequency electric energy emitted from the edge portion 102. If the edge portion 102 has an anodized coating layer 104, high-frequency electric energy is not emitted through the edge portion 102, and no operation proceeds.

In addition, the current density necessary during an operation may be maintained only when high-frequency electric energy is not emitted through the edge portion 102, and the tissue does not stick to side surfaces of the blade 101 only when the side surfaces of the blade 101 remain insulated.

In addition, a non-adhesive coating layer 105 is formed on the upper portion of the anodized coating layer 104 of the blade 101.

As the non-adhesive coating layer 105, a ceramic coating layer or Teflon coating layer may be formed.

The ceramic coating layer is formed to have a thickness of 20um to 40um.

Currently, a widely used monopolar conductive electrode is generally obtained by coating the surface of a conductive electrode made of stainless steel with glass to have a thickness of 0.15mm. A conductive electrode made of stainless steel, if coated with glass to have a thickness of 0.15mm, has appropriate insulation characteristics and non-adhesiveness such that tissue does not easily stick thereto.

If the glass coating is too thick, a crack occurs and destroys insulation. Therefore, the glass coating is formed to have a thickness of 0.15mm for the sake of appropriate insulation characteristics and non-adhesiveness.

Meanwhile, processes for processing stainless steel material and coating a blade made of stainless steel with glass are very complicated and difficult.

According to the present disclosure, an anodized coating layer 104 is formed to have a thickness of 30um to 50pm for insulation, and a ceramic coating layer is formed on the upper portion of the anodized coating layer 104 to have a thickness of 20um to 40um for non-adhesiveness.

If the thickness of the anodized coating layer 104 is less than 30um, the insulation characteristics are degraded. Therefore, the ceramic coating layer needs to be formed to have a thickness of at least 40um for insulation. If the thickness of the ceramic coating layer is equal to/larger than 40um, a crack may occur in the ceramic coating layer and destroy insulation.

In addition, if the thickness of the anodized coating layer 104 exceeds 50um, the insulation characteristics are not improved in proportion to the thickness, but the production cost alone increases. Therefore, the anodized coating layer 104 is formed to have a thickness of 30um to 50um.

If the thickness of the anodized coating layer 104 exceeds 50um, making the same thick, the ceramic coating layer may be formed thin. If the ceramic coating layer is formed below 20um, the non-adhesiveness (non-stick) performance is degraded, and tissue sticks to the surface of the blade 101 and is not easily removed.

Therefore, the anodized coating layer 104 is preferably formed to have a thickness of 30um to 50um, and the ceramic coating layer is preferably formed on the upper portion thereof to have a thickness of 20um to 40um. The best insulation characteristics and non-adhesiveness are exhibited if the anodized coating layer 104 is formed to have a thickness of 40um, and if the ceramic coating layer is formed on the upper portion thereof to have a thickness of 30um.

In addition, as the non-adhesive coating layer 105, a Teflon coating layer may be formed to have a thickness of 20um to 40um in stead of the ceramic coating layer. The reason the Teflon coating layer is formed to have the above-mentioned thickness is the same as the ceramic coating layer is formed to have a thickness of 20um to 40um.

As in FIG. 5, it is preferred to form no anodized coating layer 104 on the edge portion 102 and to form only a non-adhesive coating layer 105 thereon.

The edge portion 102 contacts tissue and emits high-frequency electric energy so as to incise and excise the tissue. By forming a ceramic coating layer or a Teflon coating layer on the edge portion 102 as the non-adhesive coating layer 105, the tissue is prevented from sticking to the edge portion 102 and thus being carbonized. It is preferred to form the non-adhesive coating layer 105 on the edge portion 102 as well such that even tissue that has adhered to the edge portion 102 can be easily removed.

When the conductive electrode 100 of the present disclosure configured as described above is fastened to the handpiece 10 and used, high-frequency electric energy supplied from the control unit 20 is supplied to the blade 101 through the plug 103 of the conductive electrode 100.

Each of both side surfaces of the blade 101 molded in a thin plate shape has an anodized coating layer 104 and a non-adhesive coating layer 105 formed thereon such that, when tissue is incised or excised with the conductive electrode 100, high-frequency electric energy is emitted only through the edge portion 102 of the blade 101, thereby incising and excising the tissue.

While the blade 101 incises tissue with high-frequency electric energy emitted from the edge portion, the incised tissue may make incomplete contact with both side surfaces of the blade 101. The blade 101 remains insulted by means of the anodized coating layer 104 such that no arc occurs due to incomplete contact with the incised tissue, and no smog is generated.

In addition, even if a part of tissue sticks to side surfaces of the blade 101 in the process of incising and excising tissue, the non-coating layer 105 makes it possible to easy remove the same.

The conductive electrode 100 of the present disclosure configured as described above is easy to process, is inexpensive, has excellent strength, and has an anodized coating layer and a non-adhesive coating layer formed on the surface thereof such that excellent insulation characteristics and non-stick characteristics are exhibited, thereby reducing contamination of the electrode due to carbonized tissue. In addition, carbonized tissue that has adhered to the conductive electrode 100 can be removed clean, burning of tissue can be reduced, and the occurrence of smog can be minimized.

The technical idea of the present disclosure has been described above with reference to above-described embodiments.

It is obvious that those skilled in the art to which the present disclosure pertains could easily modify or change the above-described embodiments in view of the description of the present disclosure.

In addition, it is obvious that, although not explicitly illustrated or described, various types of modifications, including the technical idea of the present disclosure, could be made by those skilled in the art to which the present disclosure pertains in view of the description of the present disclosure, and still fall within the scope of the present disclosure.

The above embodiments have been described with reference to the accompanying drawings for the purpose of describing the present disclosure, and the scope of the present disclosure is not limited to such embodiments.

### <Brief Description of Major Components in the Drawing>

- 10:: handpiece
- 100:: conductive electrode
- 101:: blade
- 102:: edge portion
- 103:: plug
- 104:: anodized coating layer
- 105:: non-adhesive coating layer
- 106:: stopper

## Claims

1. A conductive electrode (100) fastened to a handpiece (10) used for monopolar electrosurgery, the conductive electrode for an electrosurgical handpiece comprising:
a blade (101) molded in a long plate shape;
a plug (103) molded to extend from the blade (101) and to be inserted and fastened to the handpiece (10);
an anodized coating layer (104) formed on an outer surface of the blade (101) by performing anodized coating;
an edge portion (102) formed on a peripheral part of the blade (101) without performing anodized coating; and
a non-adhesive coating layer (105) formed by coating a surface of the anodized coating layer (104).

2. The conductive electrode for an electrosurgical handpiece of claim 1, wherein the non-adhesive coating layer (105) is a ceramic coating layer or a Teflon coating layer.

3. The conductive electrode for an electrosurgical handpiece of claim 1, wherein the anodized coating layer (104) is formed to have a thickness of 30um to 50um.

4. The conductive electrode for an electrosurgical handpiece of claim 1, wherein the non-adhesive coating layer (105) is formed to have a thickness of 20um to 40um.

5. The conductive electrode for an electrosurgical handpiece of claim 1, wherein a non-adhesive coating layer (105) is formed on a surface of the edge portion (102).

6. The conductive electrode for an electrosurgical handpiece of claim 1, wherein the blade (101) is press-molded by using aluminum as a material.

7. The conductive electrode for an electrosurgical handpiece of claim 1, wherein the edge portion (102) is formed by anodized-coating the edge portion (102) as well and then removing the anodized coating from the edge portion (102).
